# EUROPEAN PATENT SPECIFICATION

(11) **EP 2 822 480 B1**
(45) Date of publication and mention of the grant of the patent: **01.11.2017**
(21) Application number: 13714373.1
(22) Date of filing: 04.03.2013
(51) Int. Cl.: A61B 17/12, A61B 17/00

(54) **SPONGE-LIKE LEFT ATRIAL OCCLUSION DEVICE**
SCHWAMMÄHNLICHE LINKSATRIALE OKKLUSIONSVORRICHTUNG
DISPOSITIF SEMBLABLE À UNE ÉPONGE POUR OCCLUSION D'ATRIUM GAUCHE

(30) Priority: 09.03.2012 US 201261608801 P; 28.02.2013 US 201313780168
(43) Date of publication of application: 14.01.2015
(73) Proprietor: Boston Scientific Scimed, Inc., Maple Grove, MN 55311 (US)
(72) Inventor: CLARK, Christopher, St. Michael, Minnesota 55376 (US)
(74) Representative: Vossius & Partner Patentanwälte Rechtsanwälte mbB
(86) International application number: PCT/US2013/028898
(87) International publication number: WO 2013/134137

(56) References cited:
- WO-A1-2009/009466
- WO-A1-2010/081033
- US-A1- 2004 091 543
- US-A1- 2005 234 540
- US-A1- 2011 082 495

## Description

### Technical Field

This disclosure relates to devices and methods for occluding a body lumen or cavity. More particularly, the technologies disclosed herein relate to, devices, and methods for achieving atrial appendage occlusion via expandable sponge-like material.

### Background

Atrial fibrillation (AF) is the most common sustained cardiac arrhythmia affecting over 5.5 million people worldwide. AF is the irregular, chaotic beating of the upper chambers of the heart. Electrical impulses discharge so rapidly that the atrial muscle quivers or fibrillates. Episodes of AF may last a few minutes or several days. The most serious consequence of AF is ischemic stroke. It has been estimated that up to 20% of all strokes are related to AF. Most AF patients, regardless of the severity of their symptoms or frequency of episodes, require treatment to reduce the risk of stroke.

In patients with AF, blood tends to pool and form clots in an area of the heart called the left atrial appendage (LAA). The LAA is a pouch-like extension located in the upper left chamber of the heart. A blood clot that breaks loose from this area may migrate through the blood vessels and eventually occlude a smaller vessel in the brain or heart resulting in a stroke or heart attack. Clinical studies show that the majority of blood clots in patients with AF originated in the LAA.

One treatment mechanism for atrial fibrillation addresses the source of dangerous blood clots by occluding the left atrium appendage. Such treatments may include medication, surgery, or minimally invasive techniques. Surgical procedures may include trans-catheter closure of the LAA, which is challenging due to the highly variable and complex nature of human LAAs. Other closure methods include epicardial LAA ligation and surgical closure. Another approach employs metallic cages or fabric designs that occlude the ostium of the LAA when deployed. These devices are generally delivered to the treatment site via a catheter system.

There remains a need for improvement and/or alternatives in providing systems and methods for occlusion of left atrial appendage. Patent document US 2011/082495 discloses an occluder for the LAA with resilient struts as occluding elements. The invention is defined in claim 1. Further embodiments of the invention are defined in the dependent claims.

### Summary

The disclosure is directed to several alternative designs, materials, and methods of manufacturing medical device structures and assemblies.

Accordingly, some embodiments pertain to a medical device for occluding left atrial appendage of a human heart, including an occlusion device comprising an expandable sponge-like component having a proximal end and a distal end. The expandable sponge-like component of the medical device is configured to transition between a compressed configuration and an expanded configuration such that the expanded configuration is configured to conform to at least the ostium of the LAA, which can prevent clots from exiting the LAA to the left atrium of the heart. The sponge-like component may have any desired geometry such as , but not limited to, cylindrical or spherical shape. Further, the component may include a biodegradable biomaterial having a porous structure, and may also be impregnated with a therapeutic agent that is released when the occlusion device is deployed or which releases slowly over time after the occlusion device is deployed. Some embodiments may also contain a coating.

Some embodiments pertain to an occlusion system for left atrial appendage of a human heart, including a catheter with a lumen extending along the central catheter axis. The catheter has a proximal end and a distal end. The system further includes an occlusion device comprising an expandable sponge-like component that may include some or all of the features described above. The system may further include a push wire disposed within the lumen, the distal end of the push wire is in contact with the proximal end of the sponge-like component and is operable to advance the sponge-like component out of the distal end of the catheter. In addition, the system may also include a connecting member configured to detachably connect the distal end of the push wire with the proximal end of the sponge-like component. To this end, the connecting member may include, for example, a male thread at the proximal end of the push wire and a corresponding female thread attached to the proximal end of the sponge-like component. Here, the female thread may include a nut.

Some embodiments also pertain to a method for occluding the Left Atrial Appendage (LLA) of a human heart. The method may include advancing a catheter via tras-septal crossing to a position within the LAA. The catheter has a proximal end, a distal end, a lumen extending therebetween, and a medical device slidingly disposed within the lumen. Here, the medical device includes an occlusion device comprising an expandable sponge-like component configured to transition between a compressed configuration and an expanded configuration, wherein the expanded configuration is configured to conform to at least the entrance to the LAA, sufficient to prevent blood flow within the heart from entering the LAA. The occlusion device may be advanced out of the lumen of the catheter resulting in expansion of the medical device from the compressed configuration to conform to the the LAA. Here, advancing the medical device out of the lumen of the catheter includes advancing the medical device using a push wire. Further, the push wire is disposed within the lumen of the catheter such that the distal end of the push wire is connected to the proximal end of the sponge-like component. In addition, the push wire may include a connecting member for providing a detachable connection between the push wire and the sponge-like component. The method may further include retracting the catheter along with the push wire once the device is deployed.

The summary of some example embodiments in not intended to describe each disclosed embodiment or every implementation of the disclosure.

### Brief Description of the Drawings

The present disclosure may be more completely understood in consideration of the following detailed description of various embodiments in connection with the accompanying drawings, in which:
FIG. 1 illustrates a perspective view of a medical device for occluding left atrial appendage of a human heart in accordance with the present disclosure.
FIG. 2 is a schematic view of the medical device of FIG. 1 disposed within a catheter according to one embodiment of the present disclosure.
FIGS. 3 - 5 illustrate an exemplary method of using the medical device of FIG. 1 according to an embodiment of the present disclosure.

While embodiments of the present disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications, equivalents, and alternatives falling within the scope of the present disclosure.

### Detailed Description

For the following defined terms, these definitions shall be applied, unless a different definition is given in the claims or elsewhere in the specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the term "about" may be indicative as including numbers that are rounded to the nearest significant FIG.

The recitation of numerical ranges by endpoints includes all numbers within that range (e.g., 1 to 5 includes 1, 1.5, 2, 2.75, 3, 3.80, 4, and 5).

Although some suitable dimension ranges and/or values pertaining to various components, features, and/or specifications are disclosed, one of skill in the art, incited by the present disclosure, would understand desired dimensions, ranges and/or values many deviate from those expressly disclosed.

As used in this specification and the appended claims, the singular forms "a," "an," and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The detailed description and the drawings, which are not necessarily to scale, depict illustrative embodiments and are not intended to limit the scope of the disclosure. The illustrative embodiments depicted are intended only as exemplary. Selected features of any illustrative embodiment may be incorporated into an additional embodiment unless clearly stated to the contrary.

Embodiments of the present disclosure describe a medical device adapted to occlude the left atrial appendage (LAA) of a human heart. In general, the medical device may be an occlusion device comprising a compressible sponge-like component configured to transition between a compressed configuration and an expanded configuration. The expanded configuration of the sponge-like component may be configured to conform to the varying size and shape of the LAA.

While the devices and methods described herein are discussed relative to left atrial appendage occlusion, it is contemplated that the devices and methods may be used in other applications where body lumen occlusion is desired.

In some instances, it may be desirable to employ trans-septal crossing for deployment of the medical device to occlude the LAA. It may be, however, understood that other routes may also be employed to deploy the medical device within the LAA cavity. In addition, positioning the device within the LAA cavity may employ a delivery device including, but not limited to, a catheter.

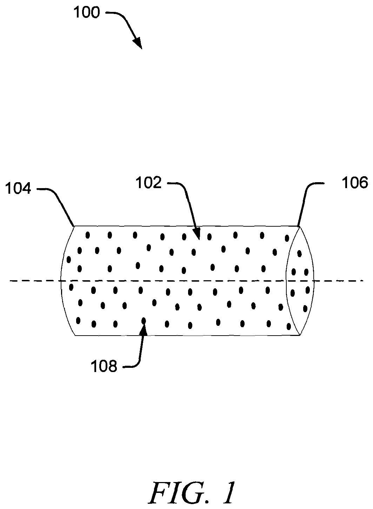

FIG. 1 illustrates an embodiment of a medical device 100 for occluding a left atrial appendage (LAA) of a human heart in accordance with the present disclosure. The medical device 100 includes an expandable sponge-like component 102 having a proximal end 104 and a distal end 106. It will be understood that "proximal" and "distal", as used in this disclosure, refers to positions or directions nearer to or farther from the user, respectively.

In the illustrated embodiment, sponge-like component 102 is cylindrical, which may also contemplate other shapes such as circular, rectangular, conical, spherical, and the like. Further, sponge-like component may have a dimension equal to or greater than the dimensions of the LAA cavity such as it may occlude the LAA. In general, the diameter of the sponge-like component 102 maybe greater than the diameter of the LAA. In addition, the configuration of the component 102 may be uniform throughout its length or it may vary based on the cavity dimensions of the LAA. The sponge-like component has a generally solid cross-section

Sponge-like component 102 may include a close-cell sponge or an open-cell sponge. The open-cell sponge-like component 102 may allow the blood flow within the heart to enter the LAA without permitting a clot formation to exit the LAA, while the close-cell sponge-like component 102 may prevent the blood flow within the heart from entering the LAA. Sponge-like component 102 also includes pores 108, which presents a porous and compressible structure to the component. In some instances, the pores 108 have pore size within the range of from about 10 to about 20 microns.

The sponge-like component 102 is a resilient compressible material, which may assist the sponge-like component 102 to conform to the varying size and shape of the LAA. The sponge-like component 102 may have sufficient porosity to allow blood to pass freely through it and yet the pores are sufficiently small to trap undesirable particles. Exemplary materials appropriate for this purpose include urethane, silicone, cellulose, or polyethylene, with urethane and polyethylene being preferred.

Here, the material employed to manufacture the sponge-like component 102 may be made from metals, polymers, biodegradable biomaterials such as polylactic acid, biodegradable polymers (biopolymers), injectable and biodegradable gels, poly(ethylene glycol) (PEG)/Polyester, magnesium-based material or other appropriate materials. In addition, the material may be elastic or resilient, which may allow the device to return to a preset expanded configuration for deployment following passage in a compressed configuration through a delivery catheter. One class of such material may include shape memory alloys such as Nitinol and the like.

In one exemplary embodiment, the sponge-like component 102 may be impregnated with a therapeutic agent or solution, which may speed up the occlusion of LAA cavity. Exemplary therapeutic agent may include heparin, or any other suitable agents known to the art. Alternatively, sponge-like component 102 may include suitable therapeutic coatings. In some embodiments, the sponge-like component may be doped with a radiopaque material such as gold or platinum.

As already discussed, the medical device 100 is compressible and resilient in nature. In general, the medical device 100 is compressed during delivery and once deployed, the medical device 100 expands to conform to the LAA cavity and occlude the ostium. For deployment purposes, the sponge-like component is compressed. Different mechanisms known to those skilled in the art may be employed for deployment. The following section describes one such mechanism using a catheter.

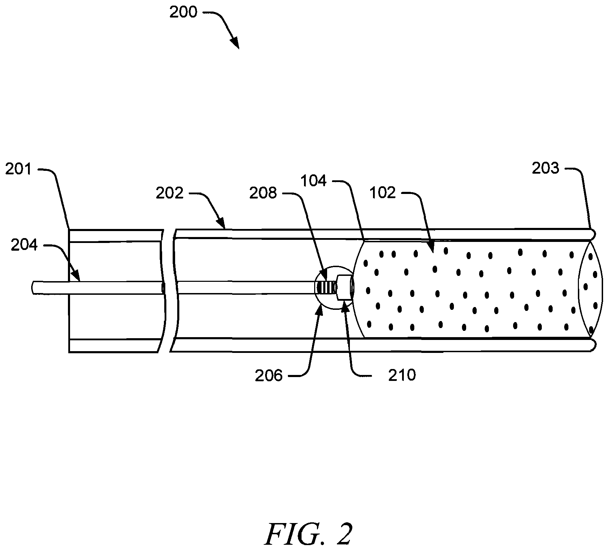

FIG. 2 illustrates the sponge-like component 102 disposed within a delivery catheter 200 according to an embodiment of the present disclosure. Catheter 200 includes a proximal end 201, a distal end 203, and a lumen 202 extending between the proximal and distal ends 201, 203. Here, the lumen 202 may be substantially circular and it may be made of any suitable biocompatible material such as polyurethane, plastic, or any other suitable material. Other suitable cross-sectional shapes such as elliptical, oval, polygonal, or irregular may also be contemplated. The diameter of the lumen may be uniform along its length or may vary. For example, the distal end of the catheter 200 may taper, allowing convenient insertion of the catheter within the body. Moreover, the lumen 202 may be flexible along its entire length or adapted for flexure along portions of its length. Alternatively, the lumen's distal end may be flexible while the remaining lumen may be rigid. Flexibility allows the lumen 202 to maneuver within the circuitous vasculature, while rigidity provides the necessary force to urge the lumen 202 forward.

In some instances, catheter 200 may require an appropriate guide wire or guiding member to deploy the medical device 100 into the patient's LAA. Therefore, the lumen 202 contains a push wire 204, which is slideably disposed within a lumen 202 of the delivery catheter 200. The push wire 204 serves to apply axial force on the compressed sponge-like component 102, which deploys the sponge like component within the LAA. Here, push wire 204 is connected removably to the proximal end 104 of the sponge-like component 102, and the physician may retract the push wire 204 inside the catheter lumen 202 once the sponge-like component 102 is deployed.

The push wire 204 may be coupled to the sponge-like component 102 using a connecting member 206. The connecting member 206 may be any suitable member that facilitates detachable connection between the two members. In one embodiment of the present disclosure, the connecting member 206 may include a male thread 208 at the proximal end of the push wire 204 and a corresponding female thread 210 such as, but not limited to, a nut attached to the proximal end of the sponge-like component 102. The connecting member may assist holding the sponge-like component 102 in the compressed configuration inside the catheter lumen 202, and it may help release the sponge-like component 102 in its expanded state once deployed. It will be understood that other mechanisms to provide a detachable connection between the push wire 204 and the sponge-like component may be contemplated. Such mechanisms may include a magnetic connection, or a snap-fit method, or any other suitable mechanism known in the art.

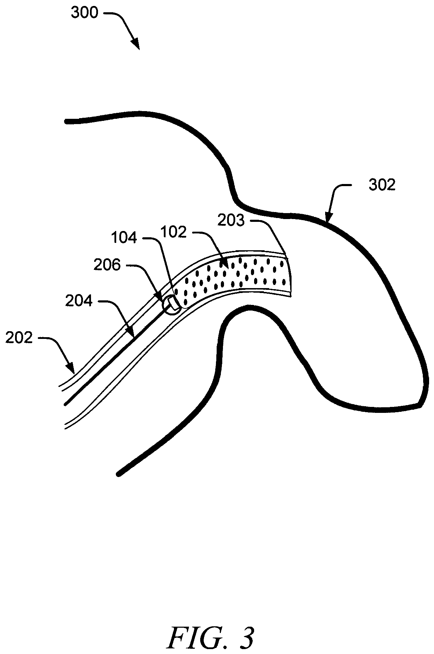

FIGS. 3-5 illustrate an exemplary method of using the medical device 100 to occlude the LAA opening 302 of a patient's heart 300.

As depicted in FIG. 3, catheter 200 is inserted within a patient's heart 300 through the trans-septal crossing. Medical device 100 may be disposed within the catheter's lumen 202 or may be inserted within the lumen 202 once the catheter's distal end reaches close to LAA opening 302. During the insertion process the medical device 100 remains compressed within the lumen 202. Once the catheter 200 is appropriately deployed, the medical device 100 extends from the distal end of the catheter 200 and expands to conform the LAA opening 302.

Deployment of the medical device 100 may be accomplished using suitable mechanism. For example, the push wire 204 may be connected to the proximal end of the medical device 100, and this push wire 204 may extend out the proximal end of the catheter 200. A surgeon may push the push wire 204 to extend the medical device 100 from the distal end of the catheter 200 or pull the catheter 200 while leaving the push wire 204 and medical device in position. In some instances, the medical device 100 may not be appropriately delivered to the LAA opening 302. In that event, push wire 204 may then be used to retract the device 100 within the catheter 200 and to reposition and redeploy it, as desired.

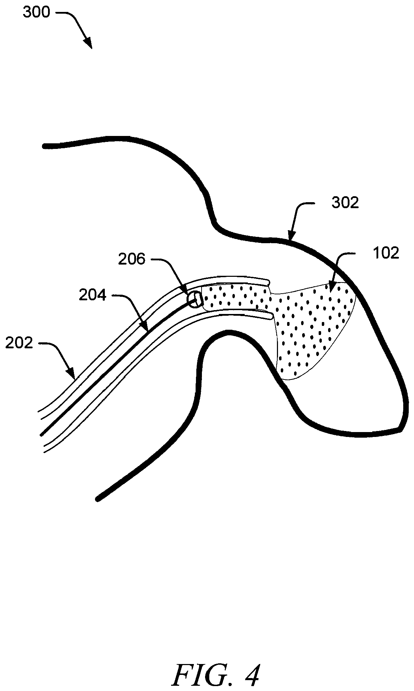

Upon medical device 100 deployment, the push wire 204 may be detached from the proximal end of the medical device 100, and the catheter 200 along with the push wire 204 may be retracted from the body. To this end, the proximal end 104 of the sponge-like component 102 may be coupled to the distal end of the push wire 204 using a connecting member 206 that facilitates detachable connection between the two devices. The connecting member may switch between a locked configuration, which may include fastening the male threads 208 over the female threads 210 to hold the device 100 within the lumen 202 and, an unlocked configuration, which may include disengagement of the two threads to releases the device 100 upon deployment. As depicted in FIG. 3, connecting member 206 may be present in a locked configuration, which includes fastening of the male threads 208 along its axis through the fixed female threads 210. Here, fastening of male threads 208 over the female threads 210 involves clockwise rotation of the male thread along its axis. Further, it may be understood that other rotation movements may also be contemplated such as anticlockwise rotation or the like. Alternatively, connecting member 206 may switch to the unlocked configuration shown in FIG. 4, which include detachment of the sponge-like component 102 and push wire 204, when deployed within the patient's LAA.

Once catheter 200 approaches the LAA opening 302, the device 100 is advanced from the distal end 203 of the catheter 200 and expands to conform to the LAA opening 302. FIG. 4 depicts this transition from a compressed configuration to an expanded configuration of sponge-like component 102. Here, the distal end 203 of the delivery catheter 200 has passed through the septum of the patient's heart 300 and is disposed adjacent the opening of the patient's LAA 302. The proximal end of the push wire 204 extends from the lumen 202 of the delivery catheter such as the proximal end of the delivery catheter is arranged to allow rotational and axial movement of the push wire 204 while preventing blood or other bodily fluids from leaking between the lumen 202 and the push wire 204.

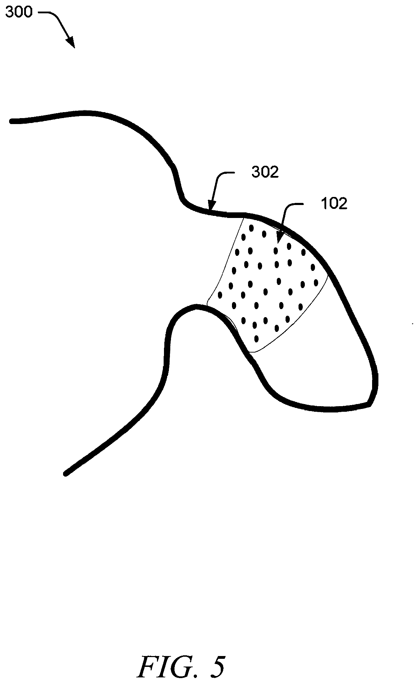

As further shown in FIG. 4, the connecting member 206 depicts unlocked configuration, which include disengaging the male threads 208 and female threads 210. Therefore, detaching the connecting member may uncouple the sponge-like component from the push wire, which may assist the partially expanded sponge-like component 102 to conform to the LAA 302 cavity, as shown in FIG. 5. Finally, the push wire 204 may be retracted within the catheter lumen 202.

Advancing the medical device 100 from the distal end of the catheter results in its expansion. The sponge-like component 102 contributes to the expandability of the medical device 100, which then expands to block the LAA opening 302. Once the medical device 100 is appropriately deployed in the LAA 302, the push wire 204 is detached from the device 100, and the catheter 200, along with the push wire 204, is retrieved from the body.

In the illustrated embodiments, the expanded sponge-like component 102 includes a rough surface, which may assist holding the sponge-like component inside the cavity by bearing against the LAA walls with sufficient force to anchor it in place. One skilled in the art may appreciate that the surface roughness of the sponge-like component 102 may be improved by including various other mechanisms known to the art. Such mechanism may include, but are not limited to, adding spikes, hooks or other components known in the art to the surface of the sponge-like component 102. In present embodiment, the force exerted on the wall of LAA 302 by the expanded sponge-like component 102 within the range of from about 180 to about 250 grams. One skilled in the art may contemplate other sizes of the sponge-like component, which may impart a force sufficient not to damage the LAA 302 wall cavity while holding the sponge-like component in position.

Those skilled in the art will recognize that the present disclosure may be manifested in a variety of forms other than the specific embodiments described and contemplated herein. Accordingly, departure in forma and detail may be made without departing from the scope of the present disclosure as described in the appended claims.

## Claims

1. A system for occluding a Left Atrial Appendage (LAA) of a human heart, the system comprising:
a catheter (200) having a proximal end (201), a distal end (203), and a lumen extending there between;
a medical device slidingly disposed within the lumen (202), the medical device including:
an expandable sponge-like component (102), having a proximal end (104) and a distal end (106) configured to transition between a compressed configuration and an expanded configuration, wherein the expanded configuration is configured to conform to at least the entrance to the LAA, sufficient to prevent blood flow within the heart from entering the LAA;
a push wire (204) disposed within the lumen, a distal end of the push wire being in contact with the proximal end (104) of the sponge-like component and operable to advance the sponge-like component out of the distal end (203) of the catheter; and
a connecting member (206) configured to detachably connect the distal end of the push wire with the proximal end of the sponge-like component.

2. The system of claim 1, wherein the sponge-like component is configured to transition to the expanded state when extended from the distal end of the catheter.

3. The system of claim 1, wherein the sponge-like component is cylindrical in shape.

4. The system of claim 1, wherein the sponge-like component includes a biodegradable biomaterial.

5. The system of claim 1, wherein the sponge-like component includes a therapeutic agent.

6. The system of claim 1, wherein the connecting member includes a male thread at the proximal end of the push wire and a corresponding female thread attached to the proximal end of the sponge-like component.

7. The system of claim 6, wherein the female thread includes a nut.

8. The system of claim 1, wherein the sponge-like component includes a coating.

## Patentansprüche

1. System zum Verschließen des linken Herzohrs (LAA) eines menschlichen Herzens, wobei das System Folgendes aufweist:
einen Katheter (200), der ein proximales Ende (201), ein distales Ende (203) und ein sich dazwischen erstreckendes Lumen aufweist,
eine medizinische Vorrichtung, die gleitend innerhalb des Lumens (202) angeordnet ist, wobei die medizinische Vorrichtung Folgendes aufweist:
eine ausdehnbare schwammähnliche Komponente (102), die ein proximales Ende (104) und ein distales Ende (106) aufweist, die dafür ausgelegt ist, zwischen einer komprimierten Konfiguration und einer ausgedehnten Konfiguration überzugehen, wobei die ausgedehnte Konfiguration dafür ausgelegt ist, zumindest dem Eingang des LAA derart zu entsprechen, dass dies ausreicht den Blutstrom innerhalb des Herzens daran zu hindern in das LAA einzutreten,
einen Schubdraht (204), der innerhalb des Lumens angeordnet ist, wobei das distale Ende des Schubdrahts in Kontakt mit dem proximalen Ende (104) der schwammähnlichen Komponente steht und dafür eingerichtet ist, die schwammähnliche Komponente aus dem distalen Ende (203) des Katheters vorzubewegen, und
ein Verbindungselement (206), das dafür ausgelegt ist, das distale Ende des Schubdrahts abnehmbar mit dem proximalen Ende der schwammähnlichen Komponente zu verbinden.

2. System nach Anspruch 1, wobei die schwammähnliche Komponente dafür ausgelegt ist, in den ausgedehnten Zustand überzugehen, wenn sie sich aus dem distalen Ende des Katheters erstreckt.

3. System nach Anspruch 1, wobei die schwammähnliche Komponente zylinderförmig ist.

4. System nach Anspruch 1, wobei die schwammähnliche Komponente ein biologisch abbaubares Biomaterial aufweist.

5. System nach Anspruch 1, wobei die schwammähnliche Komponente ein therapeutisches Mittel aufweist.

6. System nach Anspruch 1, wobei das Verbindungselement ein Außengewinde am proximalen Ende des Schubdrahts und ein entsprechendes Innengewinde, das am proximalen Ende der schwammähnlichen Komponente angebracht ist, aufweist.

7. System nach Anspruch 6, wobei das Innengewinde eine Mutter aufweist.

8. System nach Anspruch 1, wobei die schwammähnliche Komponente eine Beschichtung aufweist.

## Revendications

1. Système d'occlusion d'un appendice auriculaire gauche (AAG) d'un coeur humain, le système comprenant :
un cathéter (200) ayant une extrémité proximale (201), une extrémité distale (203), et entre lesquelles s'étend une lumière ;
un dispositif médical disposé en coulissement au sein de la lumière (202), le dispositif médical incluant :
un composant semblable à une éponge dilatable (102), ayant une extrémité proximale (104) et une extrémité distale (106) configuré pour transiter entre une configuration comprimée et une configuration dilatée, dans lequel la configuration dilatée est configurée pour se conformer à au moins l'entrée dans l'AAG, suffisante pour empêcher le flux sanguin au sein du coeur d'entrée dans l'AAG ;
un fil de poussée (204) disposé au sein de la lumière, une extrémité distale du fil de poussée étant en contact avec l'extrémité proximale (104) du composant semblable à une éponge et opérationnel pour faire avancer le composant semblable à une éponge en dehors de l'extrémité distale (203) du cathéter ; et
un organe de raccordement (206) configuré pour raccorder de façon détachable l'extrémité distale du fil de poussée avec l'extrémité proximale du composant semblable à une éponge.

2. Système selon la revendication 1, dans lequel le composant semblable à une éponge est configuré pour transiter vers l'état dilaté lorsqu'il est déployé depuis l'extrémité distale du cathéter.

3. Système selon la revendication 1, dans lequel le composant semblable à une éponge est de forme cylindrique.

4. Système selon la revendication 1, dans lequel le composant semblable à une éponge inclut un matériau biodégradable.

5. Système selon la revendication 1, dans lequel le composant semblable à une éponge inclut un agent thérapeutique.

6. Système selon la revendication 1, dans lequel l'organe de raccordement inclut un filet mâle à l'extrémité proximale du fil de poussée et un filet femelle correspondant attaché à l'extrémité proximale du composant semblable à une éponge.

7. Système selon la revendication 6, dans lequel le filet femelle inclut un écrou.

8. Système selon la revendication 1, dans lequel le composant semblable à une éponge inclut un revêtement.
